# EUROPEAN PATENT APPLICATION

(11) **EP 4 512 400 A1**
(43) Date of publication of application: **26.02.2025**
(21) Application number: 23791094.8
(22) Date of filing: 12.04.2023
(51) Int. Cl.: A61K 31/428, A61P 17/00, A61P 17/04

(54) **USE OF DITERPENE COMPOUND DERIVATIVE OR SALT THEREOF IN PREPARATION OF MEDICINE FOR PREVENTING AND TREATING ATOPIC DERMATITIS**

(30) Priority: 22.04.2022 CN 202210431695
(71) Applicant: Suzhou Pharmavan Co., Ltd., Suzhou, Jiangsu 215000 (CN)
(72) Inventor: YANG, Fei, Suzhou, Jiangsu 215000 (CN); LI, Dong, Suzhou, Jiangsu 215000 (CN); YU, Yunhui, Suzhou, Jiangsu 215000 (CN); FENG, Haimei, Suzhou, Jiangsu 215000 (CN)
(74) Representative: Plougmann Vingtoft a/s
(86) International application number: PCT/CN2023/087767
(87) International publication number: WO 2023/202439

(57) **Abstract**

The present application provides use of a diterpene compound derivative or a salt thereof in the preparation of a medicine for preventing and treating atopic dermatitis. The micromolecular diterpene compound derivative or the salt thereof has a remarkable effect of preventing and treating atopic dermatitis, shows a good prevention and treatment effect on a mouse AD model induced by OXA or MC903, can inhibit the increase of skin lesion score, the thickening of ear thickness, the aggravation of ear swelling and the increase of mast cells in mouse lesion, and also can reduce the IgE concentration of serum and improve the itching degree, indicating that the compound can be used for preventing and treating atopic dermatitis, thereby providing a new prevention and treatment thought and an effective medicine for treatment of atopic dermatitis.

## Description

### TECHNICAL FIELD

The present application belongs to the field of biomedical technology, and specifically relates to a use of a small-molecule diterpene derivative or a salt thereof in the preparation of a medicine for preventing and treating atopic dermatitis.

### BACKGROUND

Atopic dermatitis (AD) is an immune-associated, chronic, recurrent, inflammatory skin disease. It is considered to be a systemic disease because of usually comorbiding with other atopic diseases such as allergic rhinitis, asthma, etc. The clinical phenotype of AD can be greatly diverse, but is basically characterized by skin dryness, chronic eczema-like dermatitis, and intense itching.

The development of AD is closely related to genetic and environmental factors. A family history of allergic disease is the most important risk factor for the disease, and genetic factors mainly influence skin barrier function and immune homeostasis. Patients with AD often have a variety of immunological abnormalities, of which Th2 activation is an important feature, as well as skin barrier dysfunction such as reduced or absent filaggrin in the epidermis. Environmental factors include climate change, lifestyle changes, improper bathing, and infectious and allergenic stimuli. Modern lifestyles (excessive hygiene, western diet, etc.) and environmental exposures (environmental pollution, passive smoking, etc.) may be involved in the pathogenesis of AD by causing abnormalities in the immune system and skin barrier through epigenetic modifications. In addition, psychological factors (e.g., stress, anxiety, depression, etc.) also play a role in the pathogenesis of AD.

Although the pathogenesis of AD is still unclear, it has been shown by the current researches that immune abnormalities, skin barrier dysfunction, and skin flora imbalance are important factors in the pathogenesis of AD. Th2 inflammation is the basic feature of AD, and IL-4 and IL-13 are important cytokines mediating the pathogenesis of AD, which are mainly produced by Th2 cells, basophils, and type 2 innate lymphoid cells. In the chronic phase of AD, mixed inflammatory infiltrates of Th1, Th17, and Th22 cells can also be seen in the skin lesions. TSLP is produced by keratinocytes and is also an important cytokine mediating itch in AD. Skin barrier dysfunction caused by genetic mutations of Filaggrin makes the epidermis susceptible to invasion by external environmental agents (e.g. microorganisms and allergens) and initiates Th2-type inflammation, in which the Langerhans cells and dermal dendritic cells also participate through the allergen presentation. Th2-type inflammation factors inhibit the expression of proteins associated with keratinocyte barrier formation, and further disrupt the skin barrier function. AD lesions and normal-appearing skin frequently have dysbiosis of the cutaneous microbiota characterized by over-colonized *Staphylococcus aureus* and decreased flora diversity, and the resulting metabolism abnormalities promotes the progression of skin inflammation. Repeated scratching is an important factor inducing the aggravation of skin inflammation. Scratching induces the production of inflammatory mediators by keratinocytes, and also leads to the release of self-antigens and the production of IgE directed against self-antigens. Non-immune factors, such as neuroendocrine factors, may also be involved in the development of skin inflammation.

Recently, there are no drugs that can effectively cure AD.. Existing treatments only aim to improve patients' quality of life by relieving or eliminating clinical symptoms, eliminating predisposing and/or exacerbating factors, reducing and preventing relapses, and reducing or mitigating comorbidities. Despite significant advances in the treatment of AD over the past decades, the available treatment options still struggle to meet clinical needs. Clinical treatment strategies are still dominated by topical agents, oral glucocorticoids and immunosuppressive agents. Commonly used drugs include glucocorticoids (TCS), calcineurin phosphatase inhibitors (TCIs), and in recent years, approved PDE4 inhibitors, Jak inhibitors, and biologics such as Dupilumab monoclonal antibody, and other targeted agents. Anacor Pharmaceuticals developed a topical PDE4 inhibitor, Crisaborole ointment to treat mild to moderate atopic dermatitis in patients 2 years of age and older, which has been successively approved by the FDA in December 2016 (Eucrisa^{®}) and by the EMA in March 2020 (Staquis^{®}) for marketing, and permitted to import and sell in China in July 2020 (Sutamine/Staquis^{®}). Other companies are also developing topical PDE4 inhibitors, including Difamilast (OPA-15406, MM36) developed by Otsuka Pharmaceuticals, for which Otsuka Pharmaceutical is pleased to announce top-line results from two phase 3 trials conducted in Japan in patients with mild to moderate atopic dermatitis on March 27, 2020, both of which realize the primary endpoint. In January 2020, Corectim (delgocitinib) ointment, co-developed by Japan Tobacco and Torii Pharmaceutical, was approved by the Pharmaceuticals and Medical Devices Agency (PMDA) under the Ministry of Health, Labor, and Welfare of Japan for the treatment of mild to moderate AD in adults, and became the first topical JAK inhibitor for the treatment of AD in the world. At present, there are several JAK inhibitors for the AD treatment in advanced stages. First-generation JAK inhibitors target multiple JAK targets, such as Lilly's Olumiant^{®} (Baricitinib) for severe AD; second- or next-generation JAK inhibitors selectively target JAK, including Abb Vie's Rinvoq^{®} (Upadacitinib) and Pfizer's Abrocitinib for moderate and severe AD, respectively. However, marketing applications for three JAK inhibitors for AD indications have been repeatedly deferred by the FDA due to regulatory concerns about the safety of JAK inhibitors. Dupilumab injection (Dupixent^{®}), co-developed by Sanofi and Regeneron Pharmaceuticals, is a monoclonal antibody inhibiting the signalling of IL-4 and IL-13 by binding IL-4Rα subunit shared by the IL-4 and IL-13 receptor complexes, and also the first targeted biologic preparation approved by the FDA and EMA for the treatment of moderate-to-severe AD in the world. The product was approved by the NMPA on June 19, 2020 for the treatment of moderate-to-severe AD in adults (Dupixent^{®}), and on September 9 for the moderate-to-severe AD in adolescents 12 years of age and older and adults. The approval of Dupilumab marks the era of biological agent treatments for AD. In June 22, Denmark LEO Pharma announced that Adtralza (Tralokinumab), an interleukin 13 inhibitor, has been approved in the European Union for the treatment of AD indications, and that Adtralza is the first monoclonal antibody therapy specifically targeting interleukin 13 approved for the treatment of AD in the world. In July 2020, the FDA has accepted a Biologics License Application (BLA) for Tralokinumab. Tralokinumab is licensed from AstraZeneca in July 2016 by LEO Pharma. Currently, Lilly is also developing a similar drug targeting interleukin 13, Lebrikizumab, a monoclonal antibody acquired by Lilly in early 2020 through its acquisition of Dermira for a total price of approximately $1.1 billion. On August 16, Lilly announced that Lebrikizumab, its monoclonal antibody therapy targeting IL-13, has realized the primary endpoint and all key secondary endpoints in two important phase III clinical trials: ADvocate1 and ADvocate2. In addition, Maruho's Nemolizumab (licensed from Chugai Pharmaceuticals in September 2016), a humanized monoclonal antibody targeting the blockade of IL-31 receptor A, has also entered late-stage clinical trials. New drugs have been launched in the AD drug market in recent years, however, due to the safety concerns of targeted drugs and the high price of biologics, they are more likely to be used as the last treatment option, while low-cost, generic topical or oral systemic therapies are the preferred first-line drugs, and all these factors could be a huge obstacle for new drugs to enter the therapeutic market to occupy a dominant position.

Therefore, the options regarding the treatment of AD are limited in the prior art, and it is of great importance to develop more therapies for AD.

### SUMMARY

The present application provides a use of a small-molecule diterpene derivative or a salt thereof in the preparation of a medicine for preventing and treating atopic dermatitis.

In an aspect, the present application provides a use of a diterpene derivative or a salt thereof in the preparation of a medicine for preventing and treating atopic dermatitis, wherein the diterpene derivative has a structure as shown in Formula I: 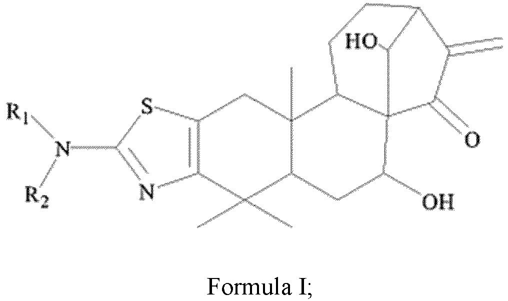 wherein R₁ and R₂ are independently substituted or unsubstituted alkyl.

The molecular structure and synthesis method of the small-molecule diterpene derivative shown in Formula I, to which the present application relates, have been disclosed in WO2018153235A1.

In some preferred embodiments of the present application, the substituted or unsubstituted alkyl is substituted or unsubstituted C1-C8 alkyl, such as C1, C2, C3, C4, C5, C6, C7 or C8 alkyl.

In the present application, a substituent of the substituted alkyl is selected from halogen-substituted C1-C5 (e.g., C1, C2, C3, C4, or C5) alkyl.

In the present application, the alkyl is linear or branched alkyl.

In some preferred embodiments, R₁ and R₂ are independently methyl, ethyl, n-propyl, isopropyl, n-butyl, tert-butyl, isobutyl, n-heptyl, n-octyl or n-hexyl.

In some more preferred embodiments, R₁ and R₂ are independently methyl, ethyl, n-propyl or isopropyl.

In one embodiment, the diterpene derivative is any one of the following compounds:

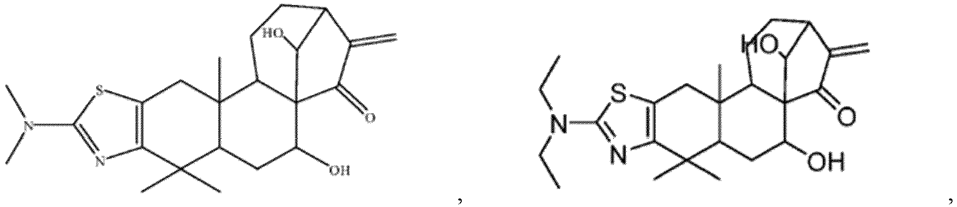

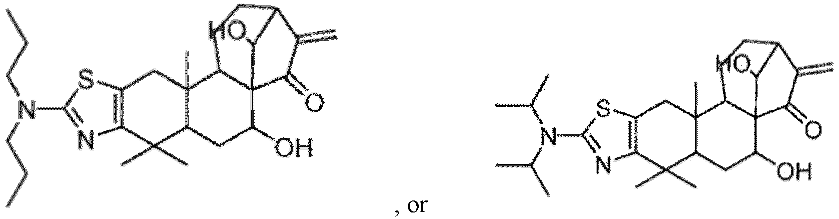

In some preferred embodiments, the salt includes tartrate, stearate, oxalate, citrate, lactate, sorbate, fumarate, formate, acetate, benzoate, benzenesulfonate, ethylsulfonate, resinate, trifluoroacetate, maleate, methanesulfonate, fumarate, amino acid salts, nicotinic acid salts, phosphate, sulfate, hydrochloride or hydrobromide.

In the present application, the salts of the diterpene derivative all have the effect of preventing and treating atopic dermatitis, and the salts of the diterpene derivative in the present application can be converted into stable free base forms by adjusting the pH value in its preparation to exert the medicinal effect, thereby effectively providing pharmaceutical efficacy of the diterpene derivative during the use process, and showing excellent therapeutic effect for atopic dermatitis.

In the present application, the diterpene derivative or the salt thereof are able to alleviate the degree of skin itching in the preventing and treating atopic dermatitis.

In the present application, with respect to the preventing and treating atopic dermatitis, the diterpene derivative or the salt thereof are capable of reducing the serum IgE concentration (e.g., reducing the serum IgE concentration in mice having atopic dermatitis).

In the present application, a dosage form of the medicine is any pharmaceutically acceptable dosage form.

In some preferred embodiments, the dosage form includes any one of suspension, granule, capsule, powder, tablet, emulsion, solution, dropping pills, injection, suppository, enema, aerosol, spray, patch or drop.

In some preferred embodiments, the medicine further includes a pharmaceutically acceptable adjuvant.

In some preferred embodiments, the adjuvant includes any one or a combination of at least two of a carrier, a diluent, an excipient, a filler, a binder, a wetting agent, a disintegrant, an emulsifier, a cosolvent, a solubilizer, an osmolality regulator, a surfactant, a coating material, a colorant, a pH regulator, an antioxidant, a bacteriostat or a buffering agent. The combination of at least two is, for example, a combination of the diluent and the excipient, a combination of the binder and the wetting agent, a combination of the emulsifier and the cosolvent, etc., and any other combinations can also be selected, which are not recited here for brevity.

In some preferred embodiments, the medicine is used to relieve or eliminate itching caused by atopic dermatitis.

Compared with the prior art, the present application has the following beneficial effects:
in the present application, it is found for the first time that the small-molecule diterpene derivative shown in Formula I or the salt thereof has significant preventive and therapeutic effects for atopic dermatitis; the diterpene derivative shown in Formula I and the salt thereof show good preventive and therapeutic effects on mouse models of AD induced by OXA or MC903, which inhibit the increase of mouse-skin lesion score, ear thickening, increased ear swelling and mast cell increase, and also reduce the serum IgE concentration and relieve the itching degree, indicating that such compound can be used to prevent and treat atopic dermatitis, and provides a new approach and effective medicines to the preventing and treating atopic dermatitis.

### BRIEF DESCRIPTION OF DRAWINGS

FIG. 1A shows the change of skin lesion score over time in the mouse model of OXA-induced AD in Example 1.
FIG. 1B shows the result of skin lesion score on day 24 in the mouse model of OXA-induced AD in Example 1.
FIG. 2A shows the change of ear thickness over time in the mouse model of OXA-induced AD in Example 1.
FIG. 2B shows the result of ear thickness on day 24 in the mouse model of OXA-induced AD in Example 1.
FIG. 3A shows the change of skin lesion score over time in the mouse model of OXA-induced AD in Example 2.
FIG. 3B shows the result of skin lesion score on day 24 in the mouse model of OXA-induced AD in Example 2.
FIG. 4A shows the change of ear thickness over time in the mouse model of OXA-induced AD in Example 2.
FIG. 4B shows the result of ear thickness on day 24 in the mouse model of OXA-induced AD in Example 2.
FIG. 5A shows the result of Ki67 positive rate from immunohistochemistry in the mouse model of OXA-induced AD in Example 2.
FIG. 5B shows the result of ICAM-1 positive rate from immunohistochemistry in the mouse model of OXA-induced AD in Example 2.
FIG. 5C shows the result of VCAM-1 positive rate from immunohistochemistry in the mouse model of OXA-induced AD in Example 2.
FIG. 6A shows the result of gray value of ICAM-1 expression from Western Blot analysis in Example 2.
FIG. 6B shows the result of gray value of VCAM-1 expression from Western Blot analysis in Example 2.
FIG. 7 shows the degree of ear swelling in the mouse model of MC903-induced AD in Example 3.
FIG. 8A shows the result of epidermal thickness in the mouse model of MC903-induced AD in Example 3.
FIG. 8B shows the result of dermal thickness in the mouse model of MC903-induced AD in Example 3.
FIG. 8C shows the result of whole ear thickness in the mouse model of MC903-induced AD in Example 3.
FIG. 9 shows the result of mast cell counting in the mouse model of MC903-induced AD in Example 3.
FIG. 10A shows the result of histopathology score in the mouse model of MC903-induced AD in Example 3.
FIG. 10B shows the result of histopathological parakeratosis score in the mouse model of MC903-induced AD in Example 3.
FIG. 10C shows the result of histopathological epidermal defect score in the mouse model of MC903-induced AD in Example 3.
FIG. 10D shows the result of histopathological dermal papilla thickening score in the mouse model of MC903-induced AD in Example 3.
FIG. 10E shows the result of histopathological capillary proliferation score in the mouse model of MC903-induced AD in Example 3.
FIG. 10F shows the result of histopathological inflammatory infiltration score in the mouse model of MC903-induced AD in Example 3.
FIG. 11 shows the result of serum IgE concentration from ELISA in Example 3.
FIG. 12 shows the result of scratching times on day 10 in the mouse model of MC903-induced AD in Example 4.++

Figure Note: *p<0.05, **p<0.01, ***p<0.001 compared with the blank control group; ^{#}p<0.05, ^{##}p<0.01, ^{###}p<0.001 compared with the model group; ^{&}p<0.05, ^{&&}p<0.01, ^{&&&}p<0.001 compared with the vehicle group.

### DETAILED DESCRIPTION

The technical solutions of the present application are further described below in terms of specific embodiments. It should be clear to those skilled in the art that the embodiments are merely an aid to understanding the present application and should not be regarded as a specific limitation on the present application.

Experimental animals involved in the following embodiments were SPF-grade BALB/c mice (≥7 weeks old, female).

The scoring criteria for skin lesions involved in the following embodiments are shown in Table 1 and the histopathological scoring criteria are shown in Table 2.

**Table 1 Scoring criteria for skin lesions**

| Score | Erythema/Hemorrhage | Scarring/Dryness | Excori ati on/Erosi on | Edema |
|---|---|---|---|---|
| 0 | None | None | None | None |
| 1 | Mild, <20% | Mild, <20% | Mild, <20% | Mild, <20% |
| 2 | Moderate, 20-60% | Moderate, 20-60% | Moderate, 20-60% | Moderate, 20-60% |
| 3 | Severe, >60% | Severe, >60% | Severe, >60% | Severe, >60% |

| | | | | |
|---|---|---|---|---|
| Note: The skin lesion score for each animal is the sum of the three index scores. | | | | |

**Table 2 Histopathological scoring criteria**

| Score | Parakerat osis | Hyperker atosis | Epidermal abscess | Epidermal defect | Dermal papilla thickening | Capillary proliferation | Inflammator y infiltration |
|---|---|---|---|---|---|---|---|
| 0 | None (-) | None (-) | None (-) | None (-) | None (-) | None (-) | None (-) |
| 1 | Very mild (+) | Very mild (+) | Very mild (+) | Very mild (+) | Very mild (+) | Very mild (+) | Very mild (+) |
| 2 | Mild (++) | Mild (++) | Mild (++) | Mild (++) | Mild (++) | Mild (++) | Mild (++) |
| 3 | Moderat e (+++) | Moderat e (+++) | Moderate (+++) | Moderate (+++) | Moderate (+++) | Moderate (+++) | Moderate (+++) |
| 4 | Heavy (++++) | Heavy (++++) | Heavy (++++) | Heavy (++++) | Heavy (++++) | Heavy (++++) | Heavy (++++) |

| | | | | | | | |
|---|---|---|---|---|---|---|---|
| Note: Each term of the criteria is scored independently. | | | | | | | |

The reagents involved in the following embodiments are Compound 1 to Compound 8, provided by SUZHOU PHARMAVAN CO., LTD (prepared by the preparation method disclosed in WO2018153235A1), and Compound 1 to Compound 8 have the molecular structures as follows:

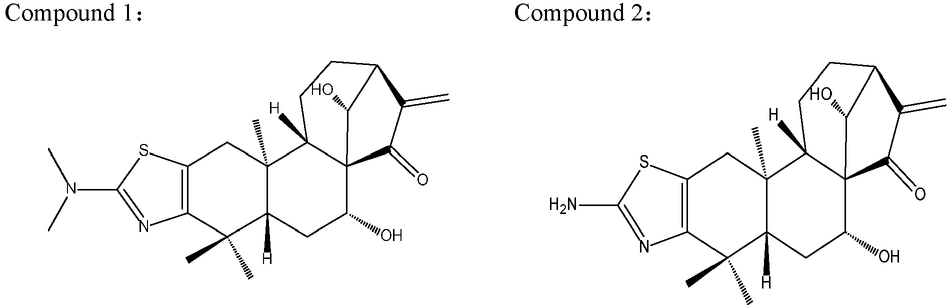

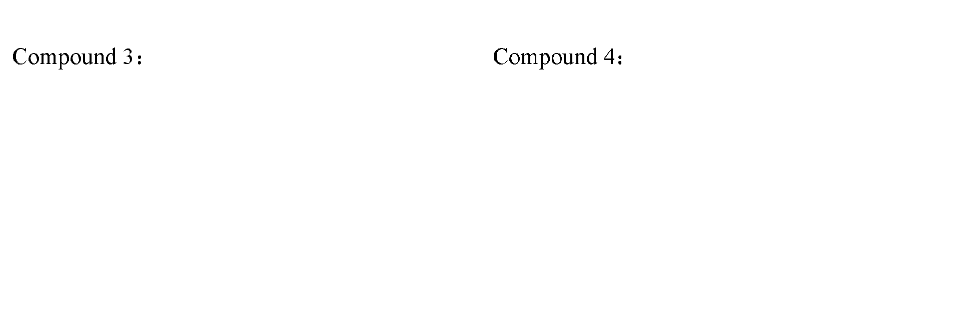

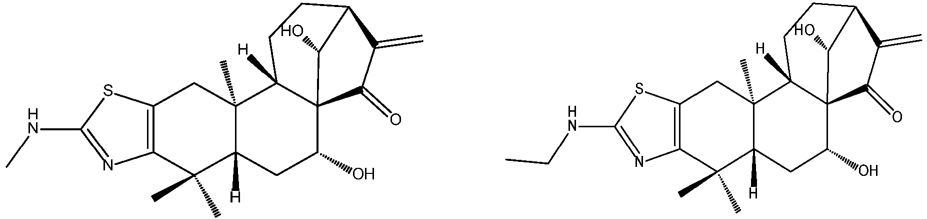

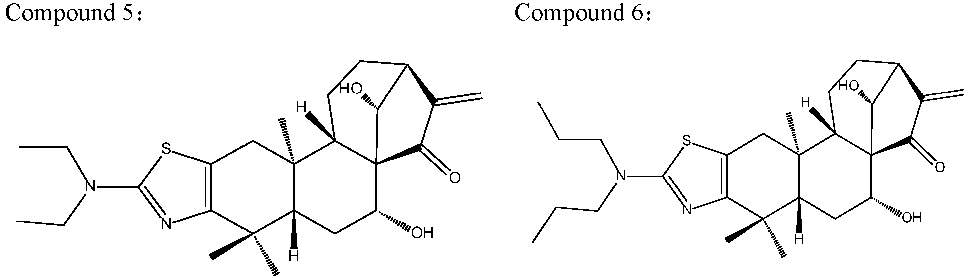

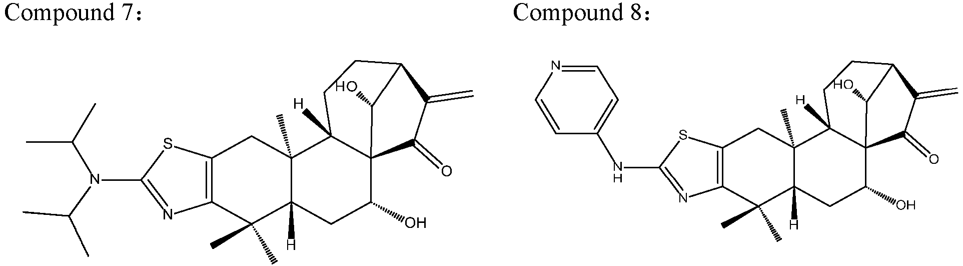

### Example 1

This example explores the preventive and therapeutic effect of eight small molecule diterpene derivatives described above on the OXA-induced AD model, including the following:
The day of grouping was defined as day 0. Mice were randomly divided into 11 groups: blank control (NC) group, model (OXA) group, blank castor oil (vehicle) group, Compound 1 administration group, Compound 2 administration group, Compound 3 administration group, Compound 4 administration group, Compound 5 administration group, Compound 6 administration group, Compound 7 administration group, and Compound 8 administration group, with 5 animals in each group. Each group except NC group was treated for modeling AD. Day 0: 0.8%-OXA solution was applied to the auricle of both ears at 20 µL per ear. Day 7, 10, 12, 14, 17, 19, 21, and 23: 0.4%-OXA solution was applied to the auricle of both ears at 20 µL per ear. Each group except NC group and OXA group was administrated. The administration started from day 7 to day 23, and the mice was administrated once a day at the auricle of both ears by 50 µL per ear (100 µL per mouse). The administration method for modeling and the design of subject dosage are shown in Table 3. The condition of the animals was observed during the experimental period, and the auricle was scored for skin lesions (see Table 1 for skin lesion scoring criteria), and the thickness of the ears was measured.

**Table 3 Treatment and dose design**

| Group | Molding reagent | Molding method | Molding volume (µL/ear) | Admini stration | Medicine concentrat ion (%) | Medicine volume (µL/ear) | Number of animals |
|---|---|---|---|---|---|---|---|
| NC group | / | / | / | / | / | / | 5 |
| OXA group | OXA | Apply to auricle | 20 | / | / | / | 5 |
| Vehicle group | OXA | Apply to auricle | 20 | Apply to auricle | / | 50 | 5 |
| Compound 1 group | OXA | Apply to auricle | 20 | Apply to auricle | 0.1 | 50 | 5 |
| Compound 2 group | OXA | Apply to auricle | 20 | Apply to auricle | 0.1 | 50 | 5 |
| Compound 3 group | OXA | Apply to auricle | 20 | Apply to auricle | 0.1 | 50 | 5 |
| Compound 4 group | OXA | Apply to auricle | 20 | Apply to auricle | 0.1 | 50 | 5 |
| Compound 5 group | OXA | Apply to auricle | 20 | Apply to auricle | 0.1 | 50 | 5 |
| Compound 6 group | OXA | Apply to auricle | 20 | Apply to auricle | 0.1 | 50 | 5 |
| Compound 7 group | OXA | Apply to auricle | 20 | Apply to auricle | 0.1 | 50 | 5 |
| Compound 8 group | OXA | Apply to auricle | 20 | Apply to auricle | 0.1 | 50 | 5 |

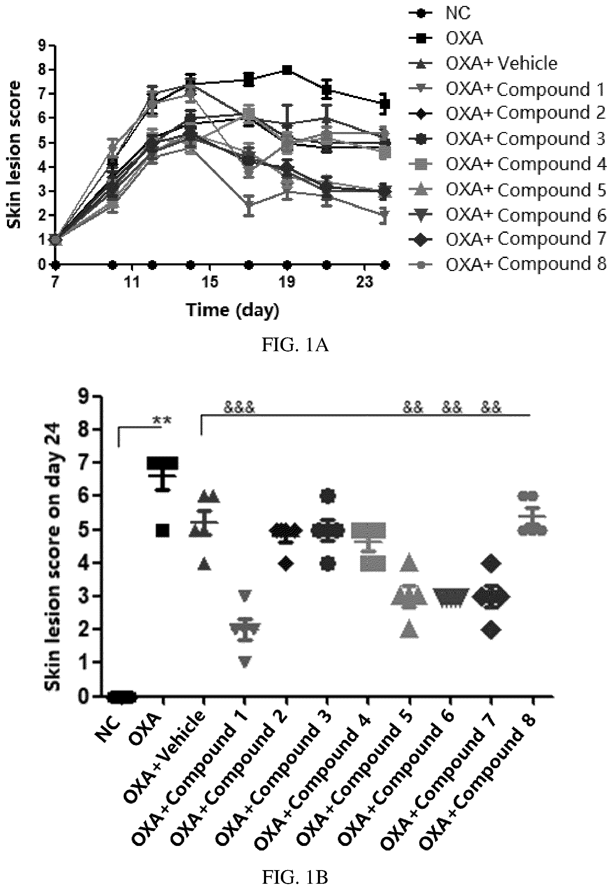

As shown in FIG. 1A and FIG. 1B, during the experiment (FIG. 1A), the skin lesion score is consistently higher in the mice of OXA group compared with the NC group. At the end of the experiment (FIG. 1B), the skin lesion scores are, in descending order, OXA group > Vehicle group > Compound 8 group > Compound 3 group > Compound 2 group > Compound 4 group > Compound 5 group = Compound 6 group = Compound 7 group > Compound 1 group > NC group. As shown by the statistical result of skin lesion scores on day 24, the OXA group is statistically different from the NC group; the groups of Compounds 1, 5, 6 and 7 are statistically different from the vehicle group. The OXA induction could significantly increase the skin lesion score of mice, and Compounds 1, 5, 6, and 7 could effectively reduce the skin lesion score.

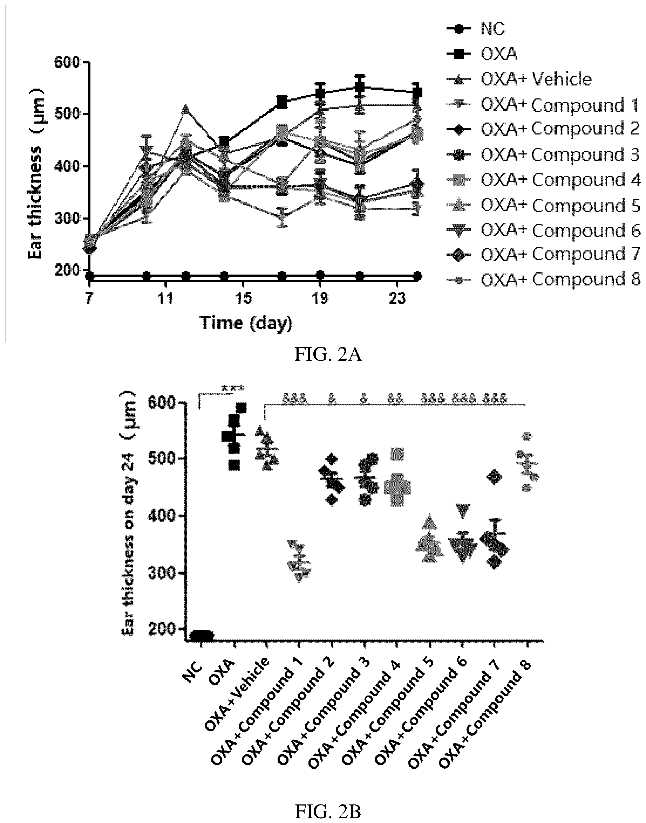

As shown in FIG. 2A and FIG. 2B, during the experiment (FIG. 2A), the right ear thickness is consistently higher in the mice of OXA group compared with the NC group. At the end of the experiment (FIG. 2B), the right ear thicknesses are, in descending order, OXA group > Vehicle group > Compound 8 group > Compound 3 group > Compound 2 group > Compound 4 group > Compound 7 group > Compound 6 group > Compound 5 group > Compound 1 group > NC group. As shown by the statistical result of right ear thickness on day 24, the OXA group is statistically different from the NC group; the groups of Compounds 1-7 are statistically different from the vehicle group. The OXA induction could significantly increase the ear thickness of mice, and Compounds 1-7 could effectively ameliorate the ear thickness.

The results of this example show that Compounds 1, 5, 6, and 7 significantly ameliorate the OXA-induced AD-like lesions in mice.

### Example 2

This example explores the preventive and therapeutic effect of a small-molecule diterpene derivative on the OXA-induced AD model, including the following:
The day of grouping was defined as day 0. Mice were randomly divided into 5 groups: blank control (NC) group, model (OXA) group, blank cream (vehicle) group, 0.1%-Compound 1 cream group, and 0.5%-Compound 1 cream group; the NC group had 5 animals, the other groups each had 10 animals. Each group except NC group was treated for modeling AD. Day 0: 0.8%-OXA solution was applied to the auricle of both ears at 20 µL per ear. Day 7, 10, 12, 14, 17, 19, 21, and 23: 0.4%-OXA solution was applied to the auricle of both ears at 20 µL per ear. Each group except NC group and OXA group was administrated. The administration started from day 7 to day 23, and the mice was administrated once a day at the auricle of both ears by 50 µL per ear (100 µL per mouse). The administration method for modeling and the design of subject dosage are shown in Table 4. The condition of the animals was observed during the experimental period, and the auricle was scored for skin lesions (see Table 1 for skin lesion scoring criteria), and the thickness of the ears was measured. The mice were euthanized, and their ear tissues were retained for immunohistochemistry and Western Blot analysis.

**Table 4 Treatment and dose design**

| Group | Molding reagent | Molding method | Molding volume (µL/ear) | Admini stration | Medicine concentrat ion (%) | Medicine volume (µL/ear) | Number of animals |
|---|---|---|---|---|---|---|---|
| NC group | / | / | / | / | / | / | 5 |
| OXA group | OXA | Apply to auricle | 20 | / | / | / | 10 |
| Vehicle group | OXA | Apply to auricle | 20 | Apply to auricle | / | 50 | 10 |
| Compoun d 1 cream group 1 | OXA | Apply to auricle | 20 | Apply to auricle | 0.1 | 50 | 10 |
| Compoun d 1 cream group 2 | OXA | Apply to auricle | 20 | Apply to auricle | 0.5 | 50 | 10 |

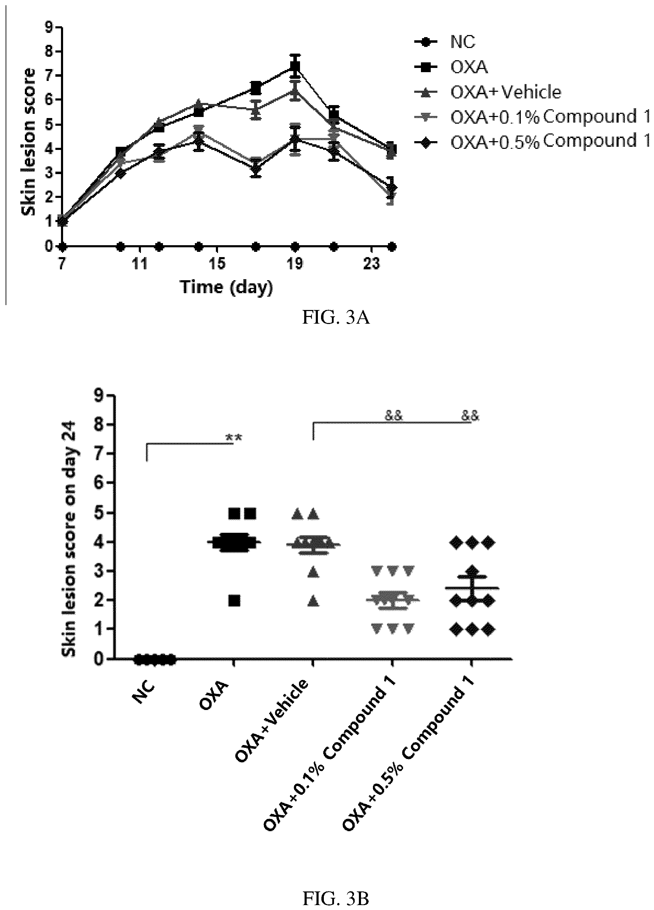

As shown in FIG. 3A and FIG. 3B, during the experiment (FIG. 3A), the skin lesion score is consistently higher in the mice of OXA group compared with the NC group. At the end of the experiment (FIG. 3B), the skin lesion scores are, in descending order, OXA group > vehicle group > 0.5%-Compound 1 cream group > 0.1%-Compound 1 cream group > NC group. As shown by the statistical result of skin lesion scores on day 24, the OXA group is statistically different from the NC group; the 0.1%-Compound 1 cream group and 0.5%-Compound 1 cream group are statistically different from the vehicle group. The OXA induction could significantly increase the skin lesion score of mice, and Compound 1 could effectively reduce the skin lesion score.

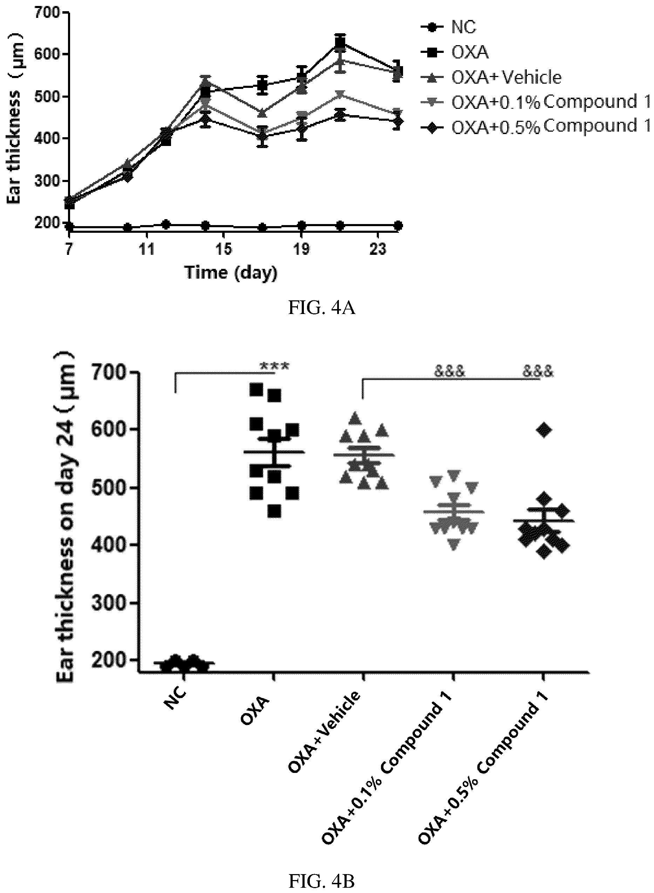

As shown in FIG. 4A and FIG. 4B, during the experiment (FIG. 4A), the right ear thickness is consistently higher in the mice of OXA group compared with the NC group. At the end of the experiment (FIG. 4B), the right ear thicknesses are, in descending order, OXA group > vehicle group > 0.1%-Compound 1 cream group > 0.5%-Compound 1 cream group > NC group. As shown by the statistical result of right ear thickness on day 24, the OXA group is statistically different from the NC group; the 0.1%-Compound 1 cream group and 0.5%-Compound 1 cream group are statistically different from the vehicle group. The OXA induction could significantly increase the ear thickness of mice, and Compound 1 could effectively ameliorate the ear thickness.

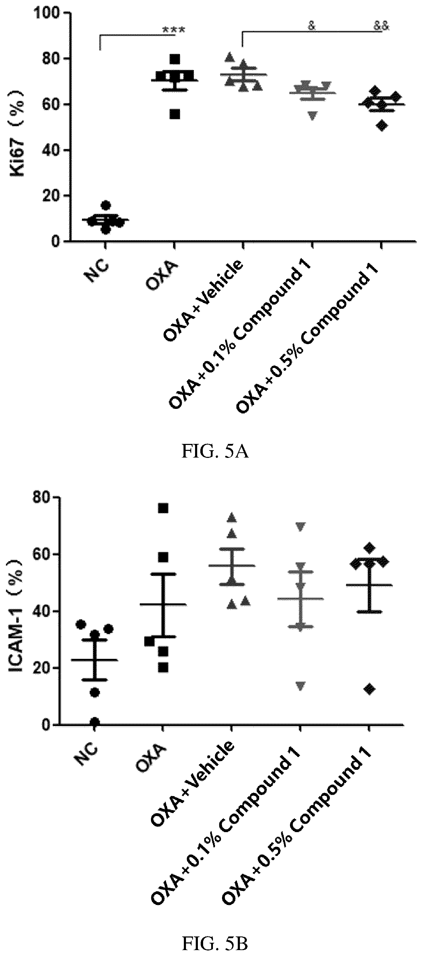

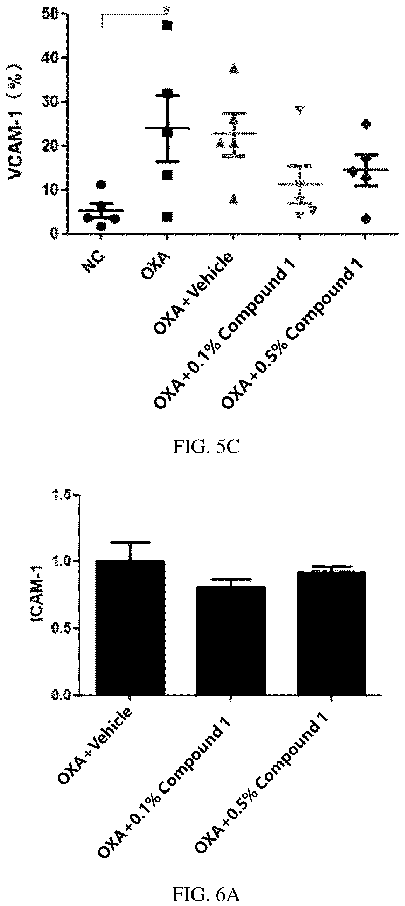

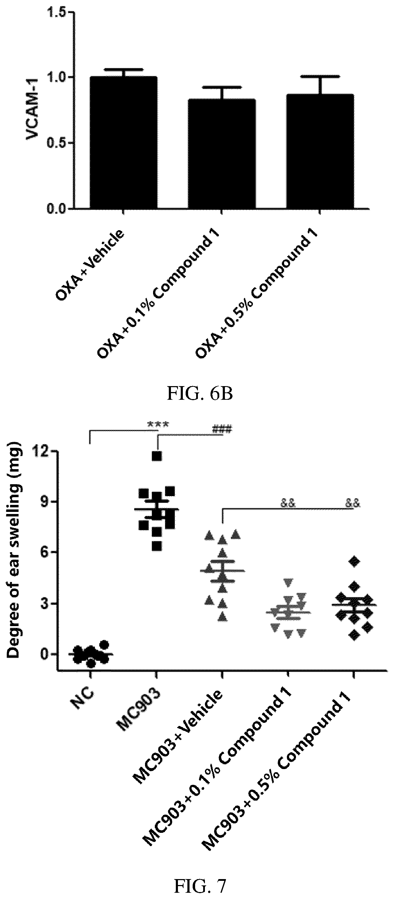

As shown in FIG. 5A- FIG. 5C, the immunohistochemical results show that in the OXA group compared with the NC group, the Ki67 positive rate is significantly increased (statistically different), and the ICAM-1 and VCAM-1 positive rates are significantly increased; in the vehicle group compared with the OXA group, the Ki67 and ICAM-1 positive rates are slightly increased and the VCAM-1 positive rate is slightly reduced; in the 0.1%-Compound 1 cream group and 0.5%-Compound 1 cream group compared with the vehicle group, the Ki67 positive rates are both reduced (statistically different), and the ICAM-1 and VCAM-1 positive rates are both reduced. As shown in FIG. 6A and FIG. 6B, ICAM-1 and VCAM-1 protein expression results detected by Western Blot analysis are consistent with the immunohistochemical results. OXA induction could accelerate the proliferation of epidermal basal cells (Ki67) and increase the adhesion molecules (ICAM-1 and VCAM-1) in skin of mice, and Compound 1 could effectively inhibit the epidermal overproliferation and aberrant expression of adhesion factor.

The results of this example show that Compound 1 significantly ameliorate the OXA-induced AD-like lesions in mice.

### Example 3

This example explores the preventive and therapeutic effect of a small-molecule diterpene derivative on the MC903-induced AD model, including the following:
The day of grouping was defined as day 0. Mice were randomly divided into 5 groups: blank control (NC) group, model (MC903) group, blank cream (vehicle) group, 0.1%-Compound 1 cream group, and 0.5%-Compound 1 cream group; each group had 10 animals. Each group except NC group was treated for modeling AD. From day 0 to day 10: 0.1 nmol/µL-MC903 solution was applied to the auricle of right ear at 20 µL once a day. Each group except NC group and MC903 group was administrated. The administration started from day 3 to day 10, and the mice was administrated once a day at the auricle of right ear by 50 µL. The administration method for modeling and the design of subject dosage are shown in Table 5. The condition of the animals was observed during the experimental period. The mice were euthanized, and their ear tissues were retained for the degree of ear swelling measurement, skin thickness measurement, mast cell counting and immunohistochemical analysis, and their blood tissues were retained for ELISA.

**Table 5 Treatment and dose design**

| Group | Molding reagent | Molding method (right ear) | Molding volume (µL) | Administrat ion (right ear) | Medicine concentr ation (%) | Medicine volume (µL) | Number of animals |
|---|---|---|---|---|---|---|---|
| NC group | / | / | / | / | / | / | 10 |
| MC903 group | MC903 | Apply to auricle | 20 | / | / | / | 10 |
| Vehicle group | MC903 | Apply to auricle | 20 | Apply to auricle | / | 50 | 10 |
| Compoun d 1 cream group 1 | MC903 | Apply to auricle | 20 | Apply to auricle | 0.1 | 50 | 10 |
| Compoun d 1 cream Group 2 | MC903 | Apply to auricle | 20 | Apply to auricle | 0.5 | 50 | 10 |

As shown in FIG. 7, MC903 induction leads to significant ear swelling in mice, and Compound 1 could effectively ameliorate the ear swelling in mice.

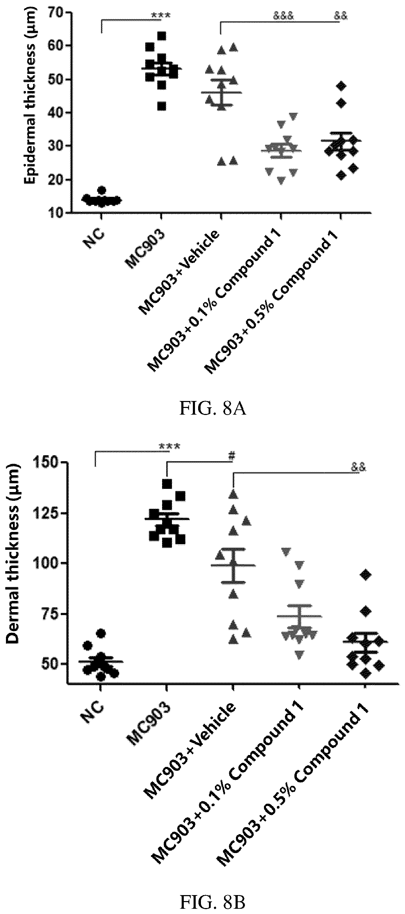

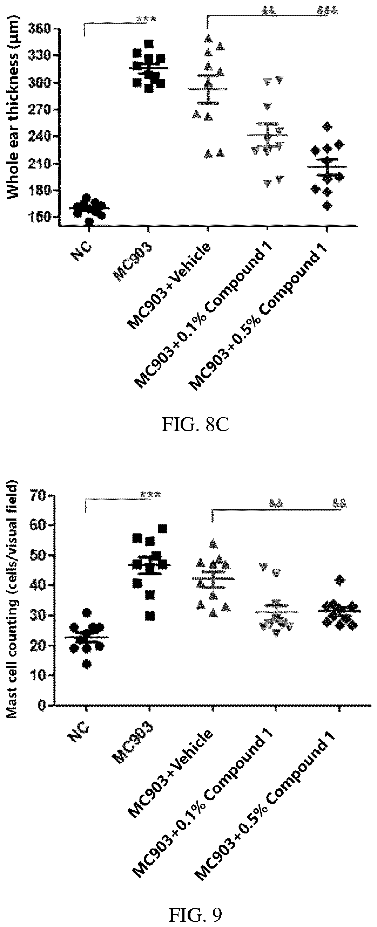

As shown in FIG. 8A, FIG. 8B and FIG. 8C, by analyzing the measurements of epidermis, dermis and whole-ear thickness of the ear histological scanning, MC903 induction leads to significant thickening of epidermis and dermis of the mouse skin, and Compound 1 effectively could effectively ameliorate the thickening of epidermis and dermis.

As shown in FIG. 9, mast cells were counted after toluidine blue staining of ear tissue sections, and the results show that Compound 1 treatment significantly reduces the number of mast cells.

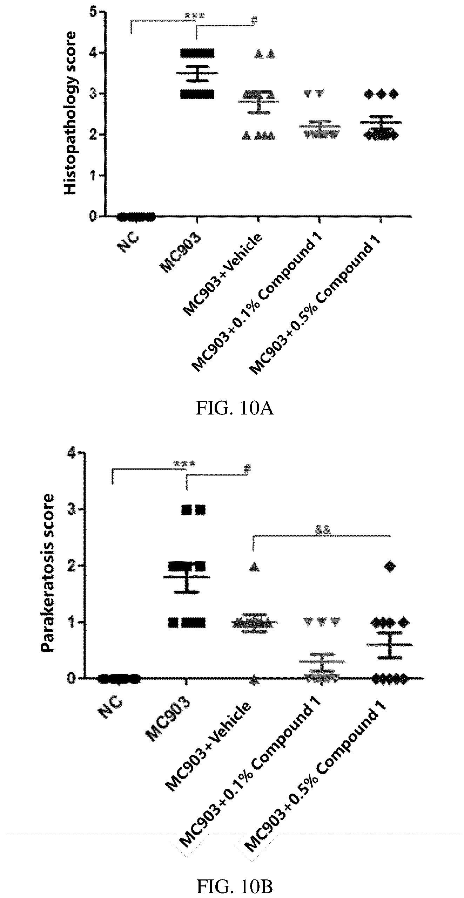

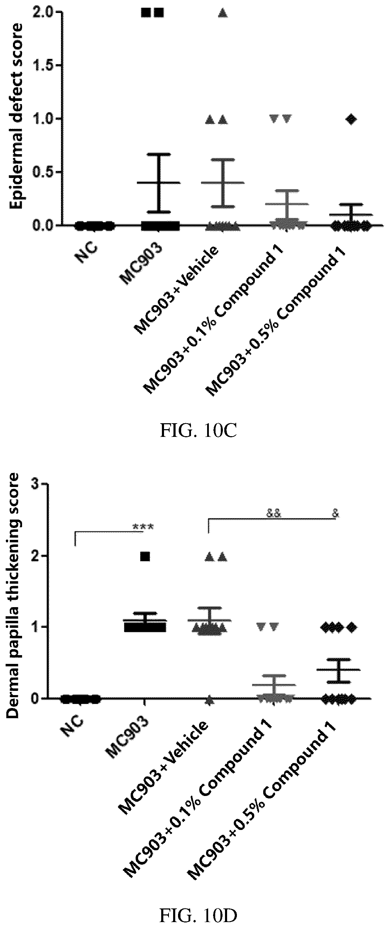

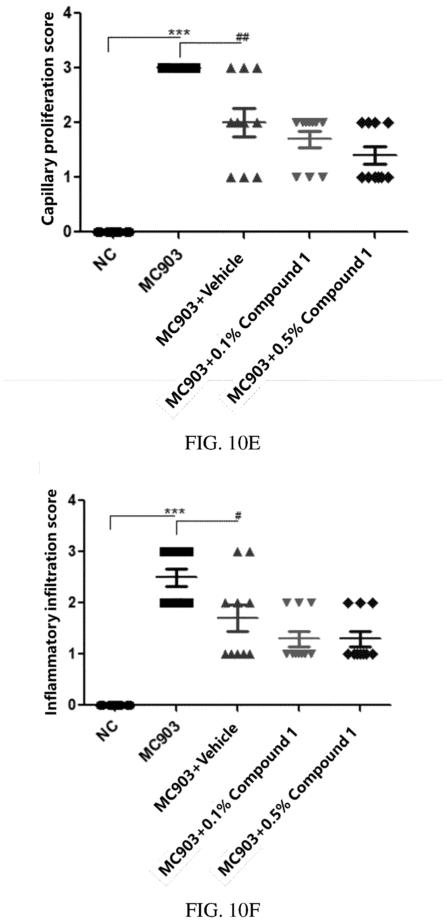

The ear tissues were subjected to HE staining, and histopathologically analyzed for the degree of lesions. After analyzing the results of the scores (see Table 2 for histopathological scoring criteria), it is found that MC903 induction leads the mice to increased skin lesions, elevated pathological scores (FIG. 10A), exacerbated parakeratosis (FIG. 10B), epidermis loss (FIG. 10C), obvious thickening of the dermal papillae (FIG. 10D), capillary proliferation in the dermis (FIG. 10E), and obvious inflammatory infiltration (FIG. 10F). Compound 1 treatment effectively ameliorates the extent of MC903-induced AD-like lesions described above.

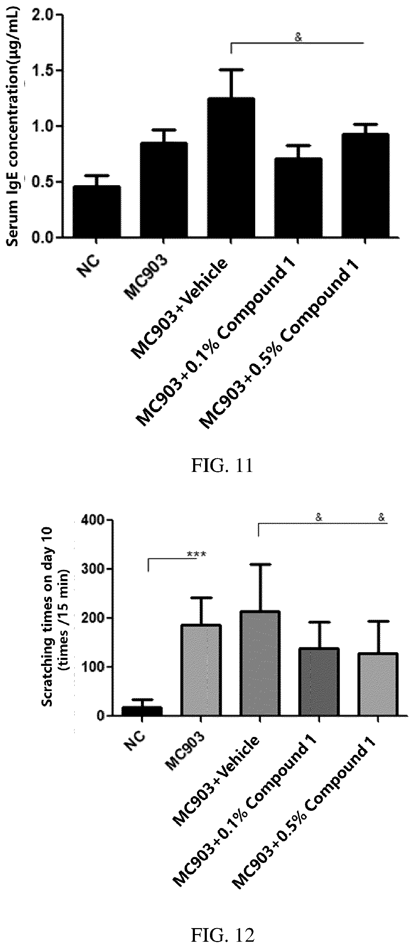

As shown in FIG. 11, the results of ELISA for serum IgE concentration show that Compound 1 inhibits the serum IgE concentration.

The results of this example show that Compound 1 significantly ameliorate the MC903-induced AD-like lesions in mice.

### Example 4

This example explores the preventive and therapeutic effect of a small-molecule diterpene derivative to itching symptoms on the MC903-induced AD model, including the following:
The day of grouping was defined as day 0. Mice were randomly divided into 5 groups: blank control (NC) group, model (MC903) group, blank cream (vehicle) group, 0.1%-Compound 1 cream group, and 0.5%-Compound 1 cream group; each group had 10 animals. Each group except NC group was treated for modeling AD. From day 0 to day 10: 0.1 nmol/µL-MC903 solution was applied to the auricle of right ear at 20 µL once a day. Each group except NC group and MC903 group was administrated. The administration started from day 3 to day 10, and the mice was administrated once a day at the auricle of right ear by 50 µL. The administration method for modeling and the design of subject dosage are same as Example 3. On the day before the experiment ended, the mice were videoed, and the mice of AD model were evaluated through the video about their scratching times in 15 min.

The results of scratching times (shown in FIG. 12) indicate that MC903 induction leads the mice to a significant increase in the scratching times and exacerbated itching symptoms, and Compound 1 treatment effectively ameliorates the extent of itching in mice.

Through the experiments in the examples, taking the reduction of skin lesion scores as the main therapeutic index, it is found in the present application for the first time that the small-molecule diterpene derivative shown in Formula I or the salt thereof is effective in prevention and treatment of atopic dermatitis, and the typical compounds such as Compound 1, Compound 5, Compound 6, and Compound 7 in the examples are structurally characterized by the bis-alkyl substitute on the thiazole amino group. Compound 2, Compound 3, Compound 4 and Compound 8 in the examples, which are structurally characterized by mono-substitution on the thiazole amino group, do not exert a significant anti-atopic dermatitis effect (taking the reduction of skin lesion scores as the main therapeutic index), probably because the mono-substitution structure weakens the affinity of the molecule for the receptor.

The applicant declares that the use of the diterpene derivative or the salt thereof in the preparation of medicines for preventing and treating atopic dermatitis is illustrated by means of the above embodiments in the present application, but the present application is not limited to the above embodiments, i.e., the present application does not necessarily rely upon the above embodiments to be implemented. It should be clear to those skilled in the art that any improvement of the present application, equivalent substitution of raw materials selected in the present application and addition of auxiliary ingredients, selection of specific methods, etc., shall fall within the scope of protection and disclosure of the present application.

## Claims

1. Use of a diterpene derivative or a salt thereof in the preparation of a medicine for preventing and treating atopic dermatitis, wherein the diterpene derivative has a structure shown in Formula I: 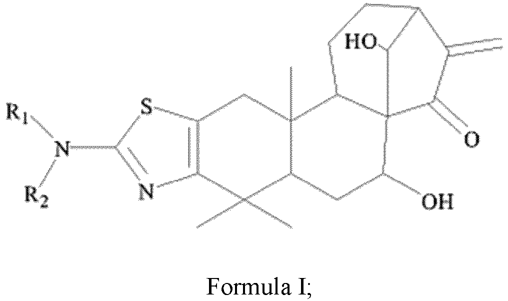 wherein R₁ and R₂ are independently substituted or unsubstituted alkyl.

2. The use according to claim 1, wherein the substituted or unsubstituted alkyl is substituted or unsubstituted C1-C8 alkyl.

3. The use according to claim 1 or 2, wherein R₁ and R₂ are independently methyl, ethyl, n-propyl, isopropyl, n-butyl, tert-butyl, isobutyl, n-heptyl, n-octyl or n-hexyl.

4. The use according to claim 3, wherein R₁ and R₂ are independently methyl, ethyl, n-propyl or isopropyl.

5. The use according to any one of claims 1-4, wherein the diterpene derivative is any one of the following compounds:

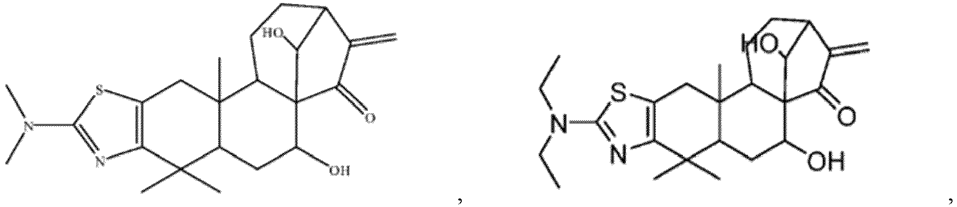

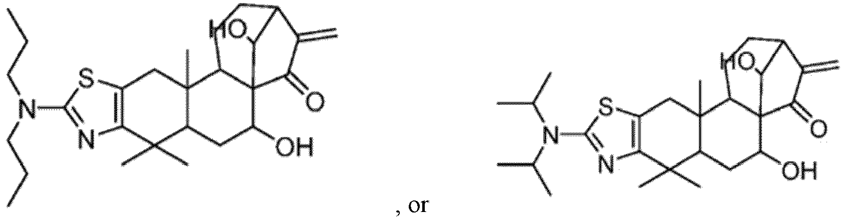

6. The use according to any one of claims 1-5, wherein the salt comprises tartrate, stearate, oxalate, citrate, lactate, sorbate, fumarate, formate, acetate, benzoate, benzenesulfonate, ethylsulfonate, resinate, trifluoroacetate, maleate, methanesulfonate, fumarate, amino acid salts, nicotinic acid salts, phosphate, sulfate, hydrochloride or hydrobromide.

7. The use according to any one of claims 1-6, wherein a dosage form of the medicine is any pharmaceutically acceptable dosage form;
preferably, the dosage form comprises any one of suspension, granule, capsule, powder, tablet, emulsion, solution, dropping pills, injection, suppository, enema, aerosol, spray, patch or drop.

8. The use according to any one of claims 1-7, wherein the medicine further comprises a pharmaceutically acceptable adjuvant.

9. The use according to claim 8, wherein the adjuvant comprises any one or a combination of at least two of a carrier, a diluent, an excipient, a filler, a binder, a wetting agent, a disintegrant, an emulsifier, a co-solvent, a solubilizer, an osmolality regulator, a surfactant, a coating material, a colorant, a pH regulator, an antioxidant, a bacteriostat or a buffering agent.

10. The use according to claim 1, wherein the medicine is used to relieve or eliminate itching caused by atopic dermatitis.
